# EUROPÄISCHE PATENTSCHRIFT

(11) **EP 1 767 226 B1**
(45) Veröffentlichungstag und Bekanntmachung des Hinweises auf die Patenterteilung: **30.03.2011**
(21) Anmeldenummer: 06024364.9
(22) Anmeldetag: 18.10.2004
(51) Int. Cl.: A61L 2/07, A61G 9/02, A61L 2/26

(54) **VORRICHTUNG ZUR REINIGUNG UND DESINFEKTION MITTELS WASSERDAMPFES VON REINIGUNGSGUT SOWIE DESSEN KÜHLUNG**
DEVICE FOR CLEANING, DISINFECTING WITH STEAM AND COOLING OF ARTICLES
DISPOSITIF POUR LE NETTOYAGE, LA DÉSINFECTION À LA VAPEUR ET LE REFROIDISSEMENT D'ARTICLES

(30) Priorität: 17.10.2003 DE 10348344
(43) Veröffentlichungstag der Anmeldung: 28.03.2007
(62) Teilanmeldung aus: 04790543.5
(73) Patentinhaber: MEIKO Maschinenbau GmbH & Co. KG, 77652 Offenburg (DE)
(72) Erfinder: Lehmann, Denis, 77799 Ortenberg (DE); Näger, Thomas, 77652 Offenburg (DE)
(74) Vertreter: Hörschler, Wolfram Johannes

(56) Entgegenhaltungen:
- EP-A2- 0 848 958
- DE-A1- 19 751 692
- DE-A1- 19 831 950
- DE-U1- 20 008 281
- US-A- 2 834 026

## Beschreibung

Die Erfindung bezieht sich auf einen Reinigungs- und Desinfektionsautomaten, welcher beispielsweise in Krankenhäusern sowie Pflegeheimen und dergleichen eingesetzt werden kann. Hier kommt es neben einer bequemen Handhabung und einer raschen Arbeitsweise auf gründliche Sauberkeit und Desinfektion des Reinigungsguts, d.h. auf Hygiene besonders an. Mittels des vorgeschlagenen Reinigungs- und Desinfektionsautomaten ist ein Verfahren zur Kühlung des Reinigungsguts durchführbar.

### Stand der Technik

Aus DE 195 09 877 C2 ist eine Vorrichtung zum Reinigen und Desinfizieren von Steckbecken, Urinflaschen und dergleichen bekannt. Diese umfasst eine Kammer, eine Dampferzeugungskammer, einen Wasservorratstank und eine Wasserpumpe, wobei eine Wassereinlassleitung in die Kammer als Überlaufleitung des Wasservorratstanks ausgebildet ist. Die Dampferzeugungskammer und der Wasservorratstank sind nach dem Prinzip kommunizierender Gefäße verbunden, derart, dass selbst bei niedrigem Wasserniveau im Wasservorratstank ein Dampferzeuger in der Dampferzeugungskammer vollständig überflutet ist und eine Dampfeinlassleitung zwischen Dampferzeugungskammer und Kammer ebenfalls eine Überlaufleitung des Wasservorratstanks und der Wasservorratstank ein Überdruckventil für die Dampfeinlassleitung bildet.

Aus DE 198 31 950 C2 ist eine Maschine zum Reinigen und Desinfizieren von Pflegegeschirr bekannt. Das Pflegegeschirr wird über eine Tür in eine Kammer eingeführt, dort gehaltert und über Düsen mit Wasser oder Dampf besprüht. Es ist ein Wasserkasten, ein Wasserzulauf und ein Pumpensystem zur Einleitung von Reinigungsflüssigkeit in eine Kammer vorgesehen. Ferner wird eine Heizeinrichtung für die Flüssigkeit und als Dampferzeuger eingesetzt, wobei der oberhalb des Flüssigkeitsspiegels des Dampfbereiches gebildete Dampf durch eine Dampfleitung in die Kammer geführt wird. Der Wasserkasten ist durch eine Trenneinrichtung bis unterhalb des niedrigsten Wasserspiegels unter Aufrechterhaltung einer Wasserverbindung in einen Hauptwasserkasten und einen Dampfbereich unterteilt. Die Heizeinrichtung ist als Dampferzeuger im Dampfbereich angeordnet.

Aus DE 198 38 180 C2 ist ein Verfahren und eine Vorrichtung zum Reinigen und Desinfizieren von Gefäßen bekannt. Mittels dieser Vorrichtung lassen sich insbesondere Steckbecken, Urinflaschen oder Absaugflaschen reinigen. Zunächst wird das betreffende Gefäß entleert und vorgespült durch kontinuierliches Besprühen mit frisch zugeführter Reinigungsflüssigkeit und kontinuierlichem Abführen verunreinigter Reinigungsflüssigkeit über einen Abfluss der Sprühvorrichtung. Das Besprühen mit Reinigungsflüssigkeit wird unterbrochen, wobei ein weiteres Abführen verunreinigter Reinigungsflüssigkeit erfolgt. Danach wird der Abfluss der Spülvorrichtung verschlossen. Es wird eine vorbestimmte Menge an Reinigungs- oder Desinfektionsflüssigkeit in die Spülkammer eingebracht. Danach erfolgt ein kontinuierliches Reinigen und Desinfizieren des Gefäßes durch Umwälzen und Besprühen mit der in der Spülkammer enthaltenden Reinigungs- oder Desinfektionsflüssigkeit. In die Spülkammer wird Heißdampf eingebracht, wobei beim Einbringen des Heißdampfes mittels einer Wasserstrahlpumpe oder einer elektrisch angetriebenen Pumpe feuchte Luft aus der Spülkammer abgesaugt wird.

Der Desinfektionsschritt erfolgt gemäß des aus DE 198 38 180 C2 bekannten Verfahrens mit Wasserdampf. Aufgrund des Einbringens von Wasserdampf in die Spülkammer heizt sich das Reinigungsgut bis zu einer eingestellten Temperatur auf. Am Ende des Desinfektionsschrittes ist die Spülkammer mit Dampf gefüllt und das Reinigungsgut auf eine Temperatur von beispielsweise 85°C aufgeheizt. Wird die Spülkammer nun durch den Bediener geöffnet, strömt der Dampf aus der Spülkammer in den Arbeitsraum. Je nach Desinfektionstemperatur, d.h. Temperatur des Wasserdampfes, besteht für den Bediener die Gefahr von Verbrühungen oder Verbrennungen durch den austretenden Dampf; ferner wird beim Öffnen der Tür der Spülkammer unnötig Feuchtigkeit in den Raum eingetragen, in dem der Reinigungs- und Desinfektionsautomat aufgestellt ist. Ferner ist es nicht ohne weiteres möglich, dass der Bediener das desinfizierte Reinigungsgut aus der Spülkammer sofort entnehmen kann, ohne sich der Gefahr einer Brandverletzung auszusetzen; es ist in der Regel eine gewisse Wartezeit erforderlich, bis das aufgeheizte Reinigungsgut durch die kühlere Raumluft abgekühlt ist.

Abhilfe wurde dadurch geschaffen, dass nach dem Desinfektionsschritt Wasser in die Spülkammer eingespritzt wird und das Waschgut auf diese Weise abgekühlt wird. Ferner wird durch das eingespritzte Wasser der Dampf in der Spülkammer kondensiert. Dieses zusätzlich eingetragene Wasser wird aus dem eingebauten Wasservorratstank des Reinigungs- und Desinfektionsautomaten entnommen. Nachteilig dabei ist, dass das Wasser aus dem Vorratstank mit Keimen belastet sein kann und damit die Gefahr einer Rückverkeimung von vorher desinfiziertem Spülgut besteht. Ein weiterer Nachteil durch die Kühlung durch nach dem Desinfektionsschritt eingetragenes Wasser liegt in einem erhöhten Wasserverbrauch. Ferner erschwert das Aufbringen des Wassers nach dem Desinfektionsschritt die selbsttätige Trocknung des aufgeheizten Reinigungsgutes.

US 2,834,026 A bezieht sich auf eine Kombination einer Spülapparatur und einer Toilettenschüssel, welche zum Reinigen von Bettpfannen und Urinalen geeignet ist. Dabei ist unter Anderem die Reinigung mit kaltem Wasser oder die Sterilisierung mit Dampf oder heißem Wasser offenbart.

DE 200 08 281 U1 offenbart eine Luftabzugsvorrichtung für WC-Schüsseln, für welche durch mehrere miteinander verbundene Kunststoffrohre eine Verbindung zwischen dem Wasserzulaufrohr und dem Wasserrohr eines WCs hergestellt wird. Die Verbindung erfolgt beispielsweise unter Umgehung des Siphonbogens.

Auch EP 0 679 406 A1 zeigt einen Reinigungsapparat zum Reinigen hohler Gegenstände, beispielsweise im medizinischen oder zahnmedizinischen Bereich, welcher einen Spülvorgang, gefolgt von einer thermischen Desinfektion des Reinigungsgutes durchführen kann. Der dargestellte Automat ist mit einer Verrohrung kleinen Durchmessers ausgestattet und auf einen geringen Wasserverbrauch ausgelegt. Nach der thermischen Desinfektion kann ein Abkühlen des Reinigungsgutes mittels komprimierter Luft erfolgen.

Die in EP 0 679 406 A1 dargestellte Vorrichtung weist jedoch verschiedene Nachteile auf. Insbesondere ist die dargestellte Vorrichtung im Wesentlichen auf die Reinigung kleiner, insbesondere zahnmedizinischer Geräte ausgelegt. Abgesehen von der Verrohrung macht sich dies beispielsweise in der Verwendung komplexer Umschaltventile bemerkbar, durch welche sowohl Flüssigkeitsabfälle, als auch, nach Umschalten, komprimierte Abluft aus der Kammer ausgeleitet werden. Derartige Schaltventile, welche sowohl für Flüssigkeiten als auch für Gase wechselweise eingesetzt werden sollen, sind aufwendig zu fertigen und komplex zu steuern. Eine Regelung allein durch in der Kammer herrschende Druckverhältnisse ohne aktive Steuerung der Ventile ist nicht möglich, so dass beispielsweise Fehlfunktionen der Ventile leicht zu einer Zerstörung der Anlage führen können. Zudem ist der Anwendungsbereich der beschriebenen Anlage aufgrund der Flüssigkeits-Gas-Ventile und der beschriebenen Verrohrung auf die Reinigung von kleinem medizinischem Gerät mittels kleiner Flüssigkeitsmengen beschränkt. Eine Reinigung von Steckbecken, Urinflaschen, Nachtgeschirr und ähnlichem, wobei in der Regel hohe Mengen flüssiger, teilweise partikelbeladener, Abwässer auftreten, ist nicht möglich.

### Darstellung der Erfindung

Angesichts der Nachteile der aus dem Stand der Technik bekannten Lösungen liegt der Erfindung die Aufgabe zugrunde, einen Reinigungs- und Desinfektionsautomaten bereitzustellen, welcher auch für die Reinigung von Reinigungsgut bei Anfall hoher Flüssigkeitsabfallmengen geeignet ist. Gleichzeitig soll die Gefahr der Rückverkeimung von desinfiziertem Reinigungsgut verringert werden, der Wasserverbrauch des Reinigungs- und Desinfektionsautomaten pro Arbeitszyklus soll möglichst gering sein, und die Arbeitsbedingungen für den Bediener des Reinigungs- und Desinfektionsautomaten sollen verbessert werden.

Erfindungsgemäß wird diese Aufgabe durch die Merkmale des Patentanspruchs 1 gelöst. Vorteilhafte Weiterbildungen sind in den Unteransprüchen dargestellt.

Es wird ein Reinigungs- und Desinfektionsautomat vorgeschlagen, welcher eine Kammer zum Aufnehmen von Reinigungsgut aufweist. Die Kammer weist ihrerseits eine Tür auf sowie einen Ablauf mit einem Siphonbogen. Weiterhin sind Mittel zum Reinigen des Reinigungsgutes mit Kaltwasser und/oder Warmwasser vorgesehen sowie Mittel zur thermischen Desinfektion des Reinigungsgutes mittels Wasserdampf und Mittel, um nach der thermischen Desinfektion zwangsweise Zuluft in die geschlossene Kammer einzubringen. Schließlich weist der Reinigungs- und Desinfektionsautomat eine Abluftleitung zum Ableiten von Abluft aus der geschlossenen Kammer bei geschlossener Tür in den Ablauf auf. Dieses Einleiten kann vorzugsweise insbesondere derart erfolgen, dass dabei ein als Sperre fungierendes Wasservolumen im Siphonbogen umgangen wird. Die Abluftleitung weist ein Abluftventil auf.

Erfindungsgemäß weisen die Mittel zum zwangsweisen Einbringen von Zuluft in die geschlossene Kammer ein Gebläse auf. Weiterhin können die Mittel zum zwangsweisen Einbringen von Zuluft in die geschlossene Kammer eine Zuluftleitung mit einem Zuluftventil aufweisen. Als Zuluft kann insbesondere Raumluft eingesetzt werden. Die Keimfreiheit der Zuluft kann zusätzlich durch einen Mikrofilter verbessert werden. Dieses Zuluftventil kann vorteilhafterweise ein selbsttätiges Absperrelement sein, welches entweder federgesteuert oder gewichtgesteuert oder membrangesteuert oder differenzdruckgesteuert oder als Rückschlagventil ausgebildet ist. Dieses selbsttätige Absperrelement kann beispielsweise als zwangsgesteuertes Absperrelement ausgebildet sein.

Weiterhin kann, alternativ oder zusätzlich, auch das Abluftventil ein selbsttätiges Absperrelement sein. Dieses kann beispielsweise entweder federgesteuert oder gewichtgesteuert oder membrangesteuert oder differenzdruckgesteuert oder als Rückschlagventil ausgebildet sein, insbesondere als zwangsgesteuertes Absperrelement ausgebildet sein.

Die Mittel zur thermischen Desinfektion des Reinigungsgutes mittels Wasserdampf können vorteilhafterweise eine Zuleitung mit einer Mündungsstelle in die Kammer aufweisen, wobei die Mündungsstelle, über welche die Kammer zeitgleich über Düsen mit Dampf beaufschlagbar ist, an einer Dachfläche der Kammer und/oder einer rückwärtigen Begrenzungswandung und/oder im unteren Bereich und/oder den seitlichen Begrenzungswänden der Kammer ausgebildet sein kann.

Die Mittel zum Reinigen des Reinigungsgutes mit Kaltwasser und/oder Warmwasser können eine Düse aufweisen. Weiterhin können diese derart ausgestaltet sein, dass dem Kaltwasser und/oder Warmwasser Hilfsstoffe zugesetzt werden können.

Die Mittel zur thermischen Desinfektion des Reinigungsgutes mittels Wasserdampf können vorteilhafterweise einen Dampferzeuger aufweisen. Es kann eine Wasser-/Dampf Einheit vorgesehen sein, aus der die Kammer sowohl mit Kaltwasser oder Warmwasser als auch mit Wasserdampf beaufschlagbar ist. Der Kaltwasser-/Warmwassereintrag in die Kammer erfolgt in diesem Fall über Sprühdüsen.

Der Reinigungs- und Desinfektionsautomat kann insbesondere eingerichtet sein, um ein Programm mit folgenden Verfahrensschritten durchzuführen:
- das Reinigungsgut wird mit Kaltwasser und/oder Warmwasser unter Zusatz von Hilfsstoffen gereinigt,
- das Reinigungsgut wird thermisch desinfiziert, und
- das Reinigungsgut wird zwangsweise abgekühlt, indem Zuluft zwangsweise in die geschlossene Kammer eingebracht wird und Abluft, zusammen mit in der Kammer enthaltenem Wasserdampf, aus der Kammer in den Ablauf abgeführt wird.

Gemäß der vorgeschlagenen Lösung laufen innerhalb des Reinigungs- und Desinfektionsautomaten die Programmschritte Spülen mit Kaltwasser, Vorreinigen mit Warmwasser (oder Kaltwasser) und Reinigung mit Klarspülung sowie thermische Desinfektion mit Wasserdampf in bekannter Weise ab. Nach der Desinfektion des Reinigungsgutes durch in die Kammer eingebrachten Wasserdampf wird erfindungsgemäß zwangsweise Luft aus der Umgebung, so zum Beispiel aus dem Arbeitsraum, in welchem der Reinigungs- und Desinfektionsautomat aufgestellt ist, in die Kammer eingeblasen. Bezogen auf die Bedingungen in der Kammer ist die Luft, die aus dem Arbeitsraum abgezogen wird, kalt und trocken. Bei Eintrag dieser Luft in die mit Wasserdampf gefüllte Kammer des Reinigungs- und Desinfektionsautomaten kondensiert der Dampf in dieser und das in der Kammer enthaltene Reinigungsgut kühlt ab. Die von außerhalb zugeführt Luft, beispielsweise Raumluft, wird zusammen mit restlichem Wasserdampf durch einen Abluftstutzen, welcher mit dem Abwassersystem des Reinigungs- und Desinfektionsautomaten verbunden sein kann, in das Abwassersystem abgeführt.

Um die Luftströmung innerhalb der Kammer des Reinigungs- und Desinfektionsautomaten zu beeinflussen, lassen sich Absperrelemente wie Schieber, Klappen und Ventile in die Leitungen von und zur Kammer einsetzen. Unter Kammer wird nachfolgend die Kammer eines Reinigungs- und Desinfektionsautomaten verstanden, in welcher das zu reinigende Gut behandelt wird. Die Absperrelemente in den Leitungen von und zur Kammer können beispielsweise als Rückschlagventil ausgebildet sein, welches zwischen dem die Luft eintragenden Gebläse und der Kammer eingesetzt werden kann. Ferner können die Absperrelemente beispielsweise ausgebildet als Klappen, Schieber oder Ventile dazu eingesetzt werden, in Betriebspausen des Reinigungs- und Desinfektionsautomaten vom Abwassersystem ausgehende Geruchsbelästigungen zu vermeiden.

Wird der in die Kammer eingetragene Luftstrom nach dem Niederschlagen des Wasserdampfes in dieser weiter aufrechterhalten, lässt sich ein zusätzlicher Effekt dahingehend erzielen, dass der eingetragene Luftstrom Feuchtigkeit von der Oberfläche des Reinigungsgutes und der Kammer aufnimmt und dieses trotz geschlossener Kammertür trocknet.

Durch das vorgeschlagene Verfahren lässt sich eine Rückverkeimung des Waschgutes vermeiden. Die in die Kammer eingeblasene Raumluft, d.h. mit Raumluftfeuchte und mit Raumlufttemperatur, ist in Bezug auf das im Wassertank vorhandene Wasser keimfrei, so dass die Gefahr einer Rückverkeimung durch im Wasser enthaltene Keime ausgeschlossen ist. Das gemäß der bisherigen Lösung zusätzlich in die Kammer eingespritzte Wasser und der damit einhergehende zusätzliche Wasserverbrauch entfällt, da zum Niederschlagen des Wasserdampfes in der Kammer ein anderes Medium, nämlich Umgebungsluft eingesetzt wird. Ferner wird durch die erfindungsgemäß vorgeschlagene Lösung weitestgehend ausgeschlossen, dass Wrasen (Dampfschwaden) in den Arbeitsbereich nach dem Öffnen der Kammertür eintreten und die Arbeitsbedingungen des den Reinigungs- und Desinfektionsautomaten betätigenden Bedieners beeinträchtigen.

Ferner ist durch die erfindungsgemäß vorgeschlagene Lösung in vorteilhafter Weise gewährleistet, dass eine Trocknung des Reinigungsgutes innerhalb der Kammer bei geschlossener Tür abläuft und somit keine zusätzliche Feuchtigkeit in den Arbeitsraum, in welchem der Reinigungs- und Desinfektionsautomat aufgestellt ist, eingetragen werden kann. Durch das zwangsweise Einblasen von Luft in die Kammer nach dem Ende des thermischen Desinfektionsschrittes wird einerseits das Niederschlagen des Wasserdampfes erreicht und andererseits eine Abkühlung des in der geschlossenen Kammer enthaltenen, gereinigten und desinfizierten Reinigungsgutes herbeigeführt. Dabei kann durch die erfindungsgemäß vorgeschlagene Lösung erreicht werden, dass das gereinigte und desinfizierte Reinigungsgut auf eine Temperatur abgekühlt wird, so dass es von der Bedienungsperson aus der Kammer entnommen werden kann, ohne das diese beim Anfassen des Reinigungsgutes Verbrennungen oder Verbrühungen erleidet.

### Zeichnung

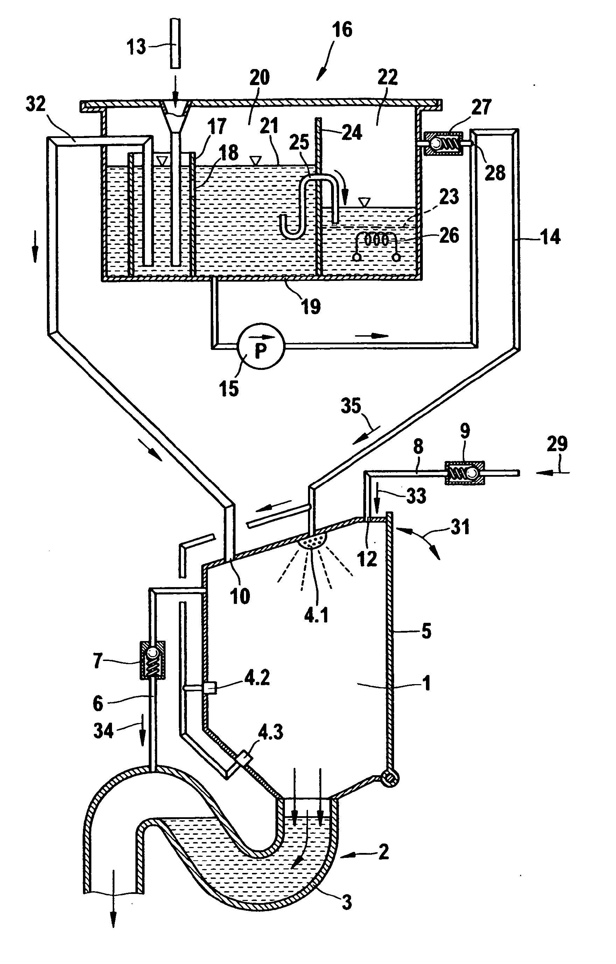

Anhand der Zeichnung wird die Erfindung nachstehend eingehender beschrieben:
Die einzige Figur zeigt in schematischer Weise den Aufbau sowie das Leitungsschema eines Reinigungs- und Desinfektionsautomaten, der mit dem erfindungsgemäß vorgeschlagenen Verfahren betreibbar ist.

In einem im Krankenhaus oder Pflegeheim eingesetzten Reinigungs- und Desinfektionsautomaten werden in einer Kammer 1 Steckbecken, Urinflaschen sowie andere Auffangbehältnisse für menschliche Ausscheidungen gereinigt und desinfiziert. Die Desinfektion dieser Behältnisse kann sowohl thermisch, d.h. mit Dampf, als auch chemisch erfolgen.

Dazu ist in den Reinigungs- und Desinfektionsautomaten in der Regel eine Kammer 1 ausgebildet, die von außen her über eine Tür 5 mit zu reinigenden Behältnissen beladen werden kann und durch welche die gereinigten desinfizierten Behältnisse aus der Kammer 1 auch wieder entnommen werden können.

Die Kammer 1 umfasst an ihrem unteren Ende einen einen Siphonbogen 3 enthaltenden Ablauf 2, über welchen die Reste menschlicher Ausscheidungen in ein Abwassersystem eingeleitet werden können. Der Ablauf 2 am unteren Ende der Kammer 1 mündet in ein Ablaufsystem. Zur Vermeidung von Geruchsbildung in der Kammer 1 dient der im Ablauf 2 ausgebildete Siphonbogen 3, der im Allgemeinen als Geruchssperre in Abwassersystemen vorgesehen ist, so auch an dem hier beschriebenen Reinigungs- und Desinfektionsautomaten. Die Kammer 1 des Reinigungs- und Desinfektionsautomaten ist durch eine schwenkbare Tür 5 zugänglich. Die Tür 5 ist um ein am unteren Ende der Tür angebrachtes Scharnier bewegbar und lässt sich in Öffnungs-/Schließrichtung 31 gemäß des in der Zeichnung eingetragenen Doppelpfeils bewegen. Die Kammer 1 ist über eine Wasser-/Dampf-Einheit 16 mit Dampf beaufschlagbar. In der Zeichnung sind Düsen 4.1, 4.2, 4.3 dargestellt, aus welchen Wasserdampf in die Kammer 1 eintreten kann. Diese sind in die Dachfläche der Kammer 1 integriert, können jedoch auch in deren seitlichen Begrenzungsflächen vorgesehen sein. Die Düsen 4.1, 4.2, 4.3, über welche Dampf in die Kammer 1 einleitbar ist, könnte ebenso gut an der rückwärtigen Begrenzungswand der Kammer 1 angebracht sein. In die Kammer 1 mündet ferner an einer Mündungsstelle 10 ein Sicherheitsüberlauf 32, über welchen Wasser aus der Wasser-/Dampf-Einheit 16 im oberen Bereich des Reinigungs- und Desinfektionsautomaten in die Kammer 1 überströmen kann und von dort in den Ablauf 2 gelangt. Der Sicherheitsüberlauf 32 könnte auch in den Ablauf 2 münden. Ferner befindet sich in der Dachfläche der Kammer 1 des Reinigungs- und Desinfektionsautomaten die Mündungsstelle 10 einer Zuluftleitung 8. Schließlich wird über eine Abluftleitung 6 Luft aus der Kammer 1 in den Ablauf 2 geführt, wobei die Mündungsstelle der Abluftleitung 6 hinter dem Siphonbogen 3 des Ablaufs 2 liegt.

Im oberen Bereich des Reinigungs- und Desinfektionsautomaten gemäß der Zeichnung ist eine Wasser-/Dampf-Einheit 16 aufgenommen. Der Wasser-/Dampf-Einheit 16 ist eine Wasserpumpe 15 zugeordnet, die unterhalb des Bodens 19 der Wasser-/Dampf-Einheit 16 angeordnet ist. Über die Wasserpumpe 15, die eine Druckerhöhung des Wassers herbeiführt, wird Wasser aus einem Wasserbehälter 20 der Wasser-/Dampf-Einheit 16 über einen Wasserzulauf 14 in die Kammer 1 gepumpt. Der Wasserbehälter 20 der Wasser-/Dampf-Einheit 16 wird über einen Kaltwasser- oder einen Warmwasserzulauf 13 versorgt. Je nach gebäudeseitigem Anschluss kann über den Wasserzulauf 13 entweder Kaltwasser in einem Temperaturbereich zwischen 10°C und 30°C in den Wasserbehälter 20 gefüllt werden oder, falls es sich um einen gebäudeseitigen Warmwasseranschluss handelt, Wasser mit einer Temperatur zwischen 45°C und 60°C in den Wasserbehälter 20 der Wasser-/Dampf-Einheit 16 einströmen. Auch eine Mischwasserbefiillung des Wasserbehälters 20 ist möglich. Das über den Wasserzulauf 13 zuströmende Warmwasser oder Kaltwasser strömt in einen Topf 17, der am Boden 19 der Wasser-/Dampf-Einheit 16 befestigt ist. Der Wasserspiegel im Topf 17 liegt im in der Zeichnung dargestellten Zustand unterhalb eines Überlaufes 18. Über den Überlauf 18 strömt das im Topf 17 über den Wasserzulauf 13 einströmende Wasser konstant in den Wasserbehälter 20 ein. Im Falle eines übermäßigen Wassereinlaufs strömt Wasser aus dem Topf 17 über den Sicherheitsüberlauf 32 durch die Mündungsstelle 10 in die Kammer 1 ein und strömt über den stets geöffneten Ablauf 2 in das Abwassersystem ab, so dass keine Wasserschäden in dem Raum auftreten können, in dem der Reinigungs- und Desinfektionsautomat aufgestellt ist.

Das aus dem Topf 17 über den Überlauf 18 in den Wasserbehälter 20 überströmende Wasser füllt den Wasserbehälter 20 bis zu einem Wasserpegel 21 an. Der Wasserbehälter 20 ist über eine Trennwand 24 von einem Dampferzeuger 22 der Wasser-/Dampf-Einheit 16 getrennt. Zur Befüllung des Dampferzeugers 20 mit Wasser erstreckt sich durch die Trennwand 24 eine Überströmleitung 25. Über die Überströmleitung 25 strömt Wasser in den Dampferzeuger 22 über. Nach dem Abpumpen stellt sich ein Pegel 23 im Dampferzeuger 22 ein. Zur Erwärmung des im Dampferzeuger 22 vorhandenen Wassers wird der im Dampferzeuger 22 enthaltene Wasservorrat über eine in der Zeichnung durch eine Wendel angedeutete Heizeinrichtung 26 erhitzt. Der bei der Erhitzung des Wassers entstehende Dampf wird über einen Leitungsabschnitt in die sich von der Wasserpumpe 15 aus erstreckende Zulaufleitung 14 zur Kammer 1 geführt. Der Leitungsabschnitt zwischen dem Dampferzeuger 22 und der Zulaufleitung 14 zur Kammer 1 ist durch ein Rückschlagventil 27 geschlossen. Aufgrund des Druckes des Wasserdampfes im Dampferzeuger 22 vermag das Rückschlagventil 27 zu öffnen, so dass an einer Mündungsstelle 28 Wasserdampf in die Zulaufleitung 14 zur Kammer 1 einströmen kann.

Über die Wasserpumpe 15 kann Wasser aus dem Wasserbehälter 20 auch über Sprühdüsen 4.2, 4.3, die im rückwandseitigen Bereich der Kammer 1 angeordnet sind, in diese eingespritzt werden. Die Sprühdüsen 4.1, 4.2, 4.3 können auch an den Seitenwänden oder an der der Kammer 1 zuweisenden Seite der die Kammer 1 verschließenden Türe angeordnet sein.

Der zur thermischen Desinfektion des in der Kammer 1 enthaltenen Reinigungsgutes benötigte Dampf wird bei entsprechender Dampferzeugung im Dampferzeuger 22 über das geöffnete Rückschlagventil 27 an der Mündungsstelle 28 in die Leitung 14 eingebracht und über diese entweder über in die Dachfläche der Kammer 1 integrierte Düsen 4.1, 4.2, 4.3 oder über an der Rückwand der Kammer 1 integrierte Düsen 4.1, 4.2, 4.3 zeitgleich in die Kammer 1 eingetragen.

Des Weiteren ist eine Belüftung der Kammer 1 über eine Zuluftleitung 8 möglich, die über ein Zuluftventil 9 freigebbar oder verschließbar ist. Über ein in der Zeichnung nicht dargestelltes Gebläse wird Zuluft 29, bei der es sich um relativ keimfreie Raumluft unter Raumluftbedingungen handelt, in die Kammer 1 eingetragen. Die Einströmrichtung der Raumluft 29 ist durch Bezugszeichen 33 kenntlich gemacht.

Das in der Kammer 1 des Reinigungs- und Desinfektionsautomaten ablaufende Programm kann gemäß der nachfolgend skizzierten Verfahrensschritte ablaufen, wobei es nicht zwingend ist, die nachfolgend aufgeführten Verfahrensschritte zu durchlaufen, sondern die nachfolgend wiedergegebenen Verfahrensschritte durchaus auch um andere Verfahrensschritte ergänzt werden können:'

Zunächst wird die Kammer 1 mit Gefäßen, wie zum Beispiel Steckbecken, Urinflaschen, Nachttöpfen oder dergleichen bestückt, die mit menschlichem Blut oder anderen menschlichen Ausscheidungen verschmutzt sind, um diese zu reinigen und wieder zu verwenden. Nach Beschicken der Kammer 1 wird die Tür 5 verschlossen und es erfolgt ein erster Spülschritt mit Kaltwasser. Über die Sprühdüsen 4.2, 4.3 werden die im Inneren der Kammer 1 aufgenommenen Behältnisse mit kaltem Wasser in einem Temperaturbereich zwischen 10°C und 30°C ausgespült. An dem genannten Ausspülschritt mit Kaltwasser kann sich entsprechend des in der Kammer 1 ablaufenden Reinigungsprogrammes ein zweiter Reinigungsschritt anschließen, der mit Warmwasser durchgeführt werden kann, wobei dieses Wasser eine Temperatur zwischen 45°C und 60°C hat. Der zweite Vorreinigungsschritt kann hingegen auch mit Kaltwasser im oben genannten Temperaturbereich durchgeführt werden, um das Ankleben von Eiweiß-Rückständen, die in den menschlichen Ausscheidungen enthalten sein können, an zu reinigendem und zu desinfizierendem Reinigungsgut zu verhindern.

An den zweiten Vorreinigungsschritt der in der Kammer 1 enthaltenen Behältnisse kann sich eine Endreinigung mit Klarspülung anschließen. Dazu kann dem Waschwasser über eine in der Zeichnung nicht dargestellte feindosierende Pumpe ein Klarspülzusatz beigemischt werden. Der Klarspülzusatz wird in geringen Mengen dem aus dem Wasserbehälter 20 über die Wasserpumpe 15 beförderten Wasser, sei es Kaltwasser, sei es Warmwasser, beigemischt und auf die durch den ersten Spülschritt und den zweiten Vorreinigungsschritt gereinigten Behältnisse aufgebracht. An den Reinigungsschritt unter Zusatz eines Klarspülmittels schließt sich ein thermischer Desinfektionsschritt an. Beim thermischen Desinfektionsschritt wird die Entnahme von kaltem oder warmem Wasser aus dem Wasserbehälter 20 der Wasser-/Dampf-Einheit 16 unterbrochen und es wird im Dampferzeuger 22 erzeugter Wasserdampf über das Rückschlagventil 27 in die Zuleitung 14 zur Kammer 1 eingeleitet. Der Wasserdampf tritt entweder an in die Dachfläche 4 der Kammer 1 integrierten Düsen 4.1, 4.2, 4.3 aus oder an Düsen 4.1, 4.2, 4.3, welche in die rückwärtige Begrenzungswand der Kammer 1 integriert sind. Über den Wasserdampf erfolgt eine Desinfektion der durch die zuvor skizzierten Reinigungsschritte gereinigten Behältnisse innerhalb der Kammer 1. Während aller vorstehend aufgezählter Schritte befindet sich die Tür 5 der Kammer 1 stets im geschlossenen Zustand. Nach dem Desinfektionsschritt ist die Kammer 1 aufgrund der geschlossenen Tür 5 vollständig mit Dampf befüllt, der nur teilweise über die Abluftleitung 6 abzuströmen vermag. Nach dem Desinfektionsschritt wird in der Zuluftleitung 8 das Zuluftventil 9 geöffnet und Raumluft 29 unter Raumluftbedingungen in die Kammer 1 eingetragen. Im Vergleich zu dem in der Kammer 1 vorhandenen Wasserdampf ist die Raumluft 29 kalt und trocken. Aufgrund dessen kondensiert der in der Kammer 1 enthaltene Wasserdampf, und das in der Kammer 1 enthaltene Reinigungsgut kühlt ab. Die zugeführte Raumluft 29 wird zusammen mit dem restlichen, in der Kammer 1 enthaltenen Wasserdampf durch die Abluftleitung 6, in welcher das Abluftventil 7 geöffnet wird, in den Ablauf 2 geleitet und so aus der Kammer 1 abgeführt. Um die Zuluftströmung in die Kammer 1 zu beeinflussen, können zusätzliche Elemente wie Schieber, Klappen, Ventile 9 oder ähnliches in der Zuluftleitung 8 sowie in der Abluftleitung 6 aufgenommen sein. Wird das Zuströmen von Raumluft 29 in Zuströmrichtung 33 in die Kammer 1 aufrechterhalten, lässt sich ein weiterer zusätzlicher Effekt dahingehend erzielen, dass der zwangsweise in die Kammer 1 eingeblasene Zuluftstrom Feuchtigkeit von der Oberfläche des Reinigungsgutes und der Waschkammer aufnimmt und die Oberfläche des Reinigungsgutes sowie der Waschkammer trotz geschlossener Tür 5 trocknet. Dadurch kann erreicht werden, dass es nach Ablauf des Waschzyklus' bei einem Öffnen der die Kammer 1 verschließenden Tür 5 nicht zu einem Entweichen von Wasserdampfschwaden kommt, welche unter ungünstigen Umständen zu Verbrühungen beziehungsweise Verbrennungen an Händen und Armen des Bedieners führen können. Es hat sich herausgestellt, dass eine Betriebszeit des in der Zuluftleitung 8 aufgenommenen Gebläses für die Raumluft 29 bzw. Luft in Kammer 1 von etwa 40 s ausreichend ist, um den in der Kammer 1 nach der thermischen Desinfektion vorhandenen Wasserdampf niederzuschlagen. Eine Verlängerung der Gebläselaufzeit in der Zuluftleitung 8 kann dazu benutzt werden, zusätzlich eine Trocknung des in der Kammer 1 enthaltenen Reinigungsgutes herbeizuführen und dieses nicht nur auf eine Temperatur abzukühlen, die dessen Handhabung erlaubt, sondern auf darunter liegende Temperaturen so z.B. unterhalb von 65°C abzukühlen. Die Zeit des in der Raumluft 29 bzw. von einer anderen Luftquelle herstammende Luft in die Kammer 1 führenden Gebläses kann je nach den Erfordernissen eingestellt werden, wobei die Laufzeit jedoch mindestens solange bemessen sein sollte, dass sichergestellt ist, dass der in der Kammer 1 enthaltene Wasserdampf vollständig niedergeschlagen ist.

Wenn auch in der Zeichnung nicht näher dargestellt, erfolgt das Zumischen des Klarspülzusatzes über eine separate Pumpe, die zusätzlich zur Wasserpumpe 15 der Wasser-/Dampf-Einheit 16 zugeordnet sein kann, wobei die Leitung, die durch diese zusätzliche Dosierpumpe beaufschlagt wird, in die Leitung 14 zur Kammer 1 mündet.

Des weiteren ist es in Abwandlung des vorstehend skizzierten Reinigungsprogrammes durchaus möglich, fest anhaftende Rückstände wie zum Beispiel Salbenrückstände, die auf Sitzflächen der menschliche Ausscheidungen aufnehmenden Behälter verbleiben können, einen Salben entfernenden Zusatz der Reinigungsflüssigkeit zuzusetzen. Neben einem zusätzlichen Programmschritt, wie z.B. dem Zusatz eines salbenentfernenden Mittels zur Reinigungsflüssigkeit, können auch zusätzliche weitere Schritte in das Reinigungsprogramm eingebaut werden, wie es auch möglich ist, die vorstehend skizzierten Reinigungsschritte je nach Erfordernissen entweder mit Kaltwasser oder mit Warmwasser in den genannten Temperaturbereichen frei zu kombinieren.

Die erfindungsgemäß vorgeschlagene Maßnahme, nach Abschluss der thermischen Desinfektion mit Wasserdampf in die Kammer 1 Raumluft 29 unter Raumluftbedingungen einzutragen, gewährleistet, dass die Gefahr der Rückverkeimung des zuvor in der Kammer 1 gereinigten und desinfizierten Reinigungsgutes durch eventuell im Wasserbehälter 20 vorhandenes unsauberes Wasser deutlich reduziert ist. Andererseits bieten die erfindungsgemäß vorgeschlagenen Maßnahmen die Möglichkeit, das in der Kammer 1 enthaltene, gereinigte und desinfizierte Reinigungsgut abzukühlen, so dass es durch den Bediener ohne weiteres aus der Kammer 1 entnommen werden kann und bei entsprechender Verlängerung der Laufzeit des in der Zuluftleitung 8 enthaltenen Gebläses kann optional eine nahezu vollständige Trocknung des in der Kammer 1 enthaltenen Reinigungsgutes erreicht werden.

Da der Wasserbehälter 20 über dem gebäudeseitig gestellten Zulauf entweder mit Kaltwasser oder mit Warmwasser befüllt wird, ist eine Verkeimung dieses Wassers nicht völlig auszuschließen. Zur Durchführung des ersten Spülschrittes beziehungsweise des zweiten Vorreinigungsschrittes ist dieses Wasser jedoch geeignet, jedoch nicht zur Herbeiführung eines Rückkühlungseffektes des Reinigungsgutes, nachdem dieses im Wege der thermischen Desinfektion mit Wasserdampf desinfiziert worden ist. Ferner bietet die erfindungsgemäß vorgeschlagene Einleitung von Raumluft 29 nach Durchführung des Desinfektionsschrittes in die Kammer 1 den Vorteil, dass eine Einsparung von bisher gemäß des Standes der Technik bekannten Lösungen zur Rückkühlung eingesetzten Wassers möglich ist. Ferner wird durch die erfindungsgemäß vorgeschlagene Lösung aufgrund der geschlossen haltbaren Tür 5 der Kammer 1 ein Austreten von Wrasen in den Arbeitsbereich des Arbeitsraumes verhindert, in welchem der Reinigungs- und Desinfektionsautomat aufgestellt ist. Des Weiteren bietet die erfindungsgemäß vorgeschlagene Einleitung von Raumluft 29 nach der Durchführung des Desinfektionsschrittes mit Wasserdampf die Möglichkeit einer Trocknung des in der Kammer 1 enthaltenen Reinigungsgutes bei geschlossener Tür 5.

### Bezugszeichenliste

- 1: Kammer
- 2: Ablauf
- 3: Siphonboden
- 4.1: Düse
- 4.2: Düse
- 4.3: Düse
- 5: Tür
- 6: Abluftleitung
- 7: Abluftventil
- 8: Zuluftleitung
- 9: Zuluftventil
- 10: Mündung Sicherheitsüberlauf

- 12: Mündung Zulufteintrag
- 13: Kaltwasser/Warmwasser-Zulauf
- 14: Zuleitung zur Kammer 1
- 15: Wasserpumpe
- 16: Wasser-/Dampf-Einheit
- 17: Topf
- 18: Überlauf
- 19: Boden
- 20: Wasserbehälter
- 21: Wasserspiegel im Wasserbehälter 20
- 22: Dampferzeuger
- 23: Wasserspiegel im Dampferzeuger
- 24: Trennwand
- 25: Überströmleitung
- 26: Heizeinrichtung
- 27: Rückschlagventil
- 28: Mündungsstelle Dampfleitung
- 29: Raumluft

- 31: Öffnungs-/Schließrichtung
- 32: Sicherheitsüberlauf
- 33: Zuströmrichtung Zuluft
- 34: Abströmrichtung Abluft
- 35: Eintrittsrichtung Wasser/Dampf

## Patentansprüche

1. Reinigungs- und Desinfektionsautomat mit einer Kammer (1) zum Aufnehmen von Reinigungsgut, wobei die Kammer eine Tür (5) aufweist, einem Ablauf (2) mit einem Siphonbogen (3), Mitteln (4.1, 4.2, 4.3, 15, 14) zum Einbringen von Kaltwasser/Warmwasser und/oder Wasserdampf, Hilfsstoffe enthaltend, umfassend Düsen (4.1, 4.2, 4.3), eine Zulaufleitung (14) sowie eine Wasserpumpe (15) zur Kammer (1) zum Reinigen des Reinigungsgutes, sowie Mitteln (4.1, 4.2, 4.3, 22, 14) zum Einbringen von Wasserdampf zur thermischen Desinfektion des Reinigungsgutes, **dadurch gekennzeichnet, dass** Mittel (8) zum zwangsweisen Einbringen von Zuluft (29) in die geschlossene Kammer (1) nach der thermischen Desinfektion, vorgesehen sind mit einem Gebläse und einer Abluftleitung (6) zum Ableiten von Abluft aus der geschlossenen Kammer (1) bei geschlossener Tür (5) in den Ablauf (2) unter Umgehung eines als Sperre fungierenden Wasservolumens im Siphonbogen (3), wobei die Abluftleitung (6) ein Abluftventil (7) aufweist, wobei das Abluftventil (7) ein Rückschlagventil ist.

2. Reinigungs- und Desinfektionsautomat gemäß dem vorhergehenden Anspruch, **dadurch gekennzeichnet, dass** die Mittel (8) zum zwangsweisen Einbringen von Zuluft (29) in die geschlossene Kammer (1) eine Zuluftleitung (8) mit einem Zuluftventil (9) aufweisen.

3. Reinigungs- und Desinfektionsautomat gemäß dem vorhergehenden Anspruch, **dadurch gekennzeichnet, dass** das Zuluftventil (9) ein selbsttätiges Absperrelement ist, welches entweder federgesteuert oder gewichtgesteuert oder membrangesteuert oder differenzdruckgesteuert ausgebildet ist oder als Rückschlagventil (7, 9) ausgeführt ist.

4. Reinigungs- und Desinfektionsautomat gemäß dem vorhergehenden Anspruch, **dadurch gekennzeichnet, dass** das selbsttätige Absperrelement als zwangsgesteuertes Absperrelement ausgebildet ist.

5. Reinigungs- und Desinfektionsautomat gemäß einem der vorhergehenden Ansprüche, **dadurch gekennzeichnet, dass** als Zuluft Raumluft eingesetzt wird.

6. Reinigungs- und Desinfektionsautomat gemäß einem der vorhergehenden Ansprüche, **dadurch gekennzeichnet, dass** die Mittel (4.1, 4.2, 4.3, 22, 14) zur thermischen Desinfektion des Reinigungsgutes mittels Wasserdampf eine Zuleitung (14) mit einer Mündungsstelle in die Kammer (1) aufweisen, wobei die Mündungsstelle, über welche die Kammer (1) zeitgleich über Düsen (4.1, 4.2, 4.3) mit Dampf beaufschlagbar ist, an einer Dachfläche der Kammer (1) und/oder einer rückwärtigen Begrenzungswandung und/oder im unteren Bereich und/oder den seitlichen Begrenzungswänden der Kammer (1) ausgebildet ist.

7. Reinigungs- und Desinfektionsautomat gemäß einem der vorhergehenden Ansprüche, **dadurch gekennzeichnet, dass** ein Mikrofilter zur Verbesserung der Keimfreiheit der Zuluft (29) vorhanden ist.

8. Reinigungs- und Desinfektionsautomat gemäß einem der vorhergehenden Ansprüche, **dadurch gekennzeichnet, dass** die Mittel (4.1, 4.2, 4.3, 14, 22) zur thermischen Desinfektion des Reinigungsgutes mittels Wasserdampf einen Dampferzeuger (22) aufweisen.

9. Reinigungs- und Desinfektionsautomat gemäß einem der vorhergehenden Ansprüche, **gekennzeichnet durch** eine Wasser-/Dampf-Einheit (16), die Kammer (1) sowohl mit Kaltwasser oder Warmwasser als auch mit Wasserdampf beaufschlagt.

10. Reinigungs- und Desinfektionsautomat gemäß einem der vorhergehenden Ansprüche, **dadurch gekennzeichnet, dass** der Reinigungs- und Desinfektionsautomat eingerichtet ist, um ein Programm mit folgenden Verfahrensschritten durchzuführen:
- das Reinigungsgut wird mit Kaltwasser und/oder Warmwasser unter Zusatz von Hilfsstoffen gereinigt,
- das Reinigungsgut wird thermisch desinfiziert, und
- das Reinigungsgut wird zwangsweise abgekühlt, indem Zuluft (29) zwangsweise in die geschlossene Kammer (1) eingebracht wird und Abluft, zusammen mit in der Kammer (1) enthaltenem Wasserdampf, aus der Kammer (1) in den Ablauf (2) abgeführt wird.

## Claims

1. Cleaning and disinfection machine having a chamber (1) for accommodating washware, wherein the chamber has a door (5), having an outlet (2) with a siphon bend (3), having means (4.1, 4.2, 4.3, 15, 14) for introducing cold water/hot water and/or steam containing additives, comprising nozzles (4.1, 4.2, 4.3), an inlet line (14) and also a water pump (15) for the chamber (1) for cleaning the washware, and also having means (4.1, 4.2, 4.3, 22, 14) for introducing steam for the thermal disinfection of the washware, **characterized in that** means (8) for the forced introduction of feed air (29) into the closed chamber (1) after thermal disinfection are provided and have a fan and a waste air line (6) for carrying away waste air from the closed chamber (1), when the door (5) is closed, into the outlet (2) so as to bypass a volume of water, which acts as a barrier, in the siphon bend (3), with the waste air line (6) having a waste air valve (7), with the waste air valve (7) being a non-return valve.

2. Cleaning and disinfection machine according to the preceding claim, **characterized in that** the means (8) for the forced introduction of feed air (29) into the closed chamber (1) have a feed air line (8) with a feed air valve (9).

3. Cleaning and disinfection machine according to the preceding claim, **characterized in that** the feed air valve (9) is an automatic shut-off element which is either spring-controlled or weight-controlled or membrane-controlled or controlled by differential pressure or is designed as a non-return valve (7, 9).

4. Cleaning and disinfection machine according to the preceding claim, **characterized in that** the automatic shut-off element is in the form of a forcibly controlled shut-off element.

5. Cleaning and disinfection machine according to one of the preceding claims, **characterized in that** room air is used as feed air.

6. Cleaning and disinfection machine according to one of the preceding claims, **characterized in that** the means (4.1, 4.2, 4.3, 22, 14) for the thermal disinfection of the washware using steam have a feed line (14) with an issue point into the chamber (1), with the issue point, by means of which steam can be supplied to the chamber (1) by means of nozzles (4.1, 4.2, 4.3) at the same time, being formed on a top face of the chamber (1) and/or a rear boundary wall and/or in the lower region and/or the lateral boundary walls of the chamber (1).

7. Cleaning and disinfection machine according to one of the preceding claims, **characterized in that** a microfilter for improving the sterility of the feed air (29) is present.

8. Cleaning and disinfection machine according to one of the preceding claims, **characterized in that** the means (4.1, 4.2, 4.3, 14, 22) for the thermal disinfection of the washware using steam have a steam generator (22).

9. Cleaning and disinfection machine according to one of the preceding claims, **characterized by** a water/steam unit (16) which supplies both cold water or hot water and also steam to the chamber (1).

10. Cleaning and disinfection machine according to one of the preceding claims, **characterized in that** the cleaning and disinfection machine is designed to carry out a program comprising the following method steps:
- the washware is cleaned with cold water and/or hot water with the addition of additives,
- the washware is thermally disinfected, and
- the washware is forcibly cooled by feed air (29) being forcibly introduced into the closed chamber (1), and waste air, together with steam which is contained in the chamber (1), being discharged from the chamber (1) into the outlet (2).

## Revendications

1. Machine automatique de nettoyage et de désinfection doté d'une chambre (1) destinée à recevoir des articles à nettoyer, ladite chambre présentant une porte (5), d'un dispositif d'écoulement (2) à coude siphon (3), de moyens (4.1, 4.2, 4.3, 15, 14) pour introduire de l'eau chaude/eau froide et/ou de la vapeur d'eau contenant des adjuvants, comprenant des buses (4.1, 4.2, 4.3), une conduite d'arrivée (14) ainsi qu'une pompe à eau (15) menant à la chambre (1) pour nettoyer les articles, ainsi que de moyens (4.1, 4.2, 4.3, 22, 14) pour introduire de la vapeur d'eau pour la désinfection thermique des articles à nettoyer, **caractérisé en ce que** des moyens (8) sont prévus permettant l'introduction forcée d'air d'arrivée (29) dans la chambre (1) fermée, après la désinfection thermique, comprenant un ventilateur et une conduite d'évacuation d'air (6) pour évacuer l'air à évacuer de la chambre (1) fermée en cas de porte (5) fermée dans le dispositif d'écoulement (2) en évitant un volume d'eau faisant fonction de barrière dans le coude siphon (3), la conduite d'évacuation d'air (6) comportant une soupape d'évacuation d'air (7), la soupape d'évacuation d'air (7) étant un clapet anti-retour.

2. Machine automatique de nettoyage et de désinfection suivant la revendication précédente, **caractérisé en ce que** les moyens (8) permettant l'introduction forcée d'air d'arrivée (29) dans la chambre (1) fermée comprennent une conduite d'amenée d'air (8) avec une soupape d'amenée d'air (9).

3. Machine automatique de nettoyage et de désinfection suivant la revendication précédente, **caractérisé en ce que** la soupape d'amenée d'air (9) est un élément d'arrêt automatique lequel est à commande par ressort ou à commande par poids ou à commande par membrane ou à commande par pression différentielle ou réalisé sous forme de clapet anti-retour.

4. Machine automatique de nettoyage et de désinfection suivant la revendication précédente, **caractérisé en ce que** l'élément d'arrêt automatique est réalisé sous forme d'élément d'arrêt à commande forcée.

5. Machine automatique de nettoyage et de désinfection suivant l'une des revendications précédentes, **caractérisé en ce que** l'on utilise comme air d'arrivée de l'air ambiant.

6. Machine automatique de nettoyage et de désinfection suivant l'une des revendications précédentes, **caractérisé en ce que** les moyens (4.1, 4.2, 4.3, 22, 14) pour la désinfection thermique des articles par vapeur d'eau comprennent une conduite d'arrivée (14) avec un point d'embouchure entrant dans la chambre (1), le point d'embouchure permettant en même temps d'alimenter la chambre (1) en vapeur par des buses (4.1, 4.2, 4.3) étant réalisé sur une surface supérieure de la chambre (1) et/ou sur une paroi d'extrémité arrière et/ou dans la partie inférieure et/ou sur les parois d'extrémité latérales de la chambre (1).

7. Machine automatique de nettoyage et de désinfection suivant l'une des revendications précédentes, **caractérisé en ce qu'**un microfiltre est présent pour améliorer l'asepsie de l'air d'arrivée (29).

8. Machine automatique de nettoyage et de désinfection suivant l'une des revendications précédentes, **caractérisé en ce que** les moyens (4.1, 4.2, 4.3, 14, 22) pour la désinfection thermique des articles à nettoyer par vapeur d'eau comprennent un générateur de vapeur (22).

9. Machine automatique de nettoyage et de désinfection suivant l'une des revendications précédentes, **caractérisé par** une unité eau/vapeur (16) alimentant la chambre (1) aussi bien en eau froide ou eau chaude qu'en vapeur d'eau.

10. Machine automatique de nettoyage et de désinfection suivant l'une des revendications précédentes, **caractérisé en ce que** la machine automatique de nettoyage et de désinfection est configuré pour effectuer un programme comprenant les étapes de procédé suivantes :
- les articles à nettoyer sont lavés à l'eau froide et/ou l'eau chaude avec addition d'adjuvants,
- les articles à nettoyer font l'objet d'une désinfection thermique, et
- les articles à nettoyer font l'objet d'un refroidissement forcé, par introduction forcée d'air d'arrivée (29) dans la chambre (1) fermée et évacuation d'air à évacuer, ensemble avec la vapeur d'eau contenue dans la chambre (1), de la chambre (1) dans le dispositif d'écoulement (2).
